(19) Europäisches Patentamt
European Patent Office
Office européen des brevets

(11) Veröffentlichungsnummer: **0 205 019**
**B1**

(12) **EUROPÄISCHE PATENTSCHRIFT**

(45) Veröffentlichungstag der Patentschrift: **18.04.90**

(51) Int. Cl.[5]: **C 07 C 17/22**, C 07 C 41/22, C 07 C 209/86, C 07 C 245/20

(21) Anmeldenummer: **86107100.9**

(22) Anmeldetag: **26.05.86**

(54) **Verfahren zur kontinuierlichen Herstellung eines kernfluorierten aromatischen Kohlenwasserstoffes.**

(30) Priorität: **07.06.85 DE 3520316**

(43) Veröffentlichungstag der Anmeldung:
**17.12.86 Patentblatt 86/51**

(45) Bekanntmachung des Hinweises auf die Patenterteilung:
**18.04.90 Patentblatt 90/16**

(84) Benannte Vertragsstaaten:
**DE FR GB IT**

(56) Entgegenhaltungen:
**US-A-2 861 984**

(73) Patentinhaber: **RIEDEL-DE HAEN AKTIENGESELLSCHAFT**
**Wunstorfer Strasse 40**
**D-3016 Seelze 1 (DE)**

(72) Erfinder: **Begemann, Eike, Dr.**
**Seekamp 4**
**D-3008 Garbsen 1 (DE)**
Erfinder: **Schmand, Horst, Dr.**
**Kramerstrasse 3**
**D-3052 Bad Nenndorf (DE)**

(74) Vertreter: **Urbach, Hans-Georg, Dr. et al**
**p.A. CASSELLA AKTIENGESELLSCHAFT**
**Postfach**
**D-6000 Frankfurt am Main 61 (DE)**

**EP 0 205 019 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung bezieht sich auf ein Verfahren zur kontinuierlichen Herstelung von kernfluorierten aromatischen Kohlenwasserstoffen durch Diazotierung von aromatischen Aminen in Gegenwart von Fluorwasserstoff und anschließende Dediazonierung der erhaltenen Aryldiazoniumfluoride in Gegenwart von Fluorwasserstoff.

Es ist bekannt, daß aromatische, im Kern fluorierte Verbindungen durch Diazotieren von aromatischen Aminen und Zersetzen der resultierenden Diazoniumverbindungen jeweils in Gegenwart von überschüssigem, wasserfreiem oder nahezu wasserfreiem Fluorwasserstoff hergestellt werde (deutsche Patentschrift 600 706; referiert in Chem. Abstracts *28*, Seite 7260 (1934)). Die Reaktionen werden in einem Eintopfverfahren durchgeführt; im einzelnen ist die Herstellung von Fluorbenzol mit einer Ausbeute von 87 Prozent und von m-Fluortoluol mit einer Ausbeute von 82 Prozent beschrieben. Die Diazotierung erfolgt bei einer Temperatur von etwa 5°C und die Zersetzung bei einer Temperatur von 30 bis 40°C.

Die vorerwähnte Arbeitsweise ist ebenfalls geeignet für die Herstellung weiterer substituierter Fluorbenzole; hierbei liegen die Ausbeuten zwischen 39 und 98 Prozent (J. Amer. Chem. Soc. *72*, 4809—4810 (1950)).

Ferner ist bekannt, daß Fluorbenzole oder Fluorpyridine durch Diazotieren von Benzolen oder Pyridinen, die ein oder zwei Aminogruppen aufweisen, und anschließendes Zersetzen der jeweiligen Diazoniumfluoride hergestellt werden, wobei beide Reaktionen in wasserfreiem Fluorwasserstoff oder in maximal 70 prozentigem, wäßrigen Fluorwasserstoff in Gegenwart von Ammoniumionen durchgeführt werden (deutsche Patentschrift 27 32 604 = US-Patentschrift 4 075 252). Die Diazotierung und Zersetzung wird entweder getrennt oder in einer Stufe ausgeführt; die Diazotierungstemperature liegt im Bereich von −20 bis +50°C und die Zersetzungstemperatur im Bereich von 15 bis 350°C. Die Reinheit der Produkte beträgt bis zu 99,9 Prozent.

Aufgabe der Erfindung ist die Herstellung von kernfluorierten, aromatischen Kohlenwasserstoffen unter Verwendung von aromatischen Aminen als Ausgangsmaterial in einem verfahrenstechnisch einfachen, kontinuierlich in technischem Maßstab sicher durchführbaren Verfahren.

Die Erfindung betrifft nun ein Verfahren zur kontinuierlichen Herstellung eines kernfluorierten aromatischen Kohlenwasserstoffes durch Diazotierung eines aromatischen Amins in Gegenwart von Fluorwasserstoff und anschließende Dediazonierung des erhaltenen Aryldiazoniumfluorids in Gegenwart von Fluorwasserstoff, das dadurch gekennzeichnet ist, daß zunächst ein aromatisches Amin in einem Schlaufenreaktor mit wasserfreiem Fluorwasserstoff bei einer Temperatur von 0 bis 15°C zu dem entsprechenden Aniliniumfluorid umgesetzt wird, wobei das Molverhältnis von Amin zu Fluorwasserstoff 1:12 bis 1:20 beträgt, dann das Aniliniumfluorid in einem weiteren Schlaufenreaktor bei einer Temperatur von 0 bis 10°C mit einem Alkalinitrit zu einem entsprechenden Aryldiazoniumfluorid umgesetzt wird, wobei das Molverhälnis von Aniliniumfluorid zu Alkalinitrit 1:1 bis 1:1,15 begrägt, und schließlich das Aryldiazoniumfluorid in einem Rieselfilmreaktor bei einer Temperatur von 60 bis 140°C zu dem entsprechenden kernfluorierten aromatischen Kohlenwasserstoff zersetzt wird.

Als Ausgangsmaterial für das erfindungsgemäße Verfahren dient ein aromatisches Amin, vorzugsweise eine Verbindung der Formel

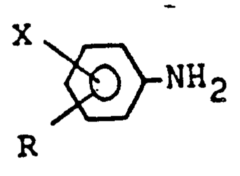

in der X ein Wasserstoffatom oder ein Halogenatom, vorzugsweise ein Chlor- oder Bromatom, bedeutet und R einen niederen Alkylrest mit vorzugsweise 1 bis 4 Kohlenstoffatomen oder einen niederen Alkoxyrest mit vorzugsweise 1 bis 4 Kohlenstoffatomen oder einen Benzylrest, der gegebenenfalls mit einer Aminogroupe substituiert ist, oder X darstellt. Geeignete aromatische Amine sind beispielsweise Anilin und substituierte Aniline wie 2-Methylanilin, 2-Ethylanilin, 3-Chloranilin, 3-Bromanilin, 3-Methoxyanilin, 3-Ethoxyanilin, 2,3-Dichloranilin, 2,3-Disbromanilin, 3,5-Dichloranilin, 3,5-Dibromanilin, 3-Chlor-2-methylanilin, 2-Amino-4-fluortoluol, 3-Fluor-6-methylanilin, 3-Chlor-4-methylanilin und 4,4′-Diaminodiphenylmethan.

Das erfindungsgemäße Verfahren wird in drei Stufen durchgeführt: In der ersten Stufe wird ein aromatisches Amin mit Fluorwasserstoff zu einem Aniliniumfluorid umgesetzt (Salzbildung), in der zweiten Stufe wird das Aniliniumfluorid mit einem Alkalinitrit zu einem Aryldiazoniumfluorid umgesetzt (Diazotierung) und in der dritten Stufe wird das Aryldiazoniumfluorid zersetzt (Dediazonierung). Alle Verfahrensstufen werden kontinuierlich in bestimmten Reaktionsgefäßen durchgeführt.

Die Salzbildung aus aromatischem Amin und Fluorwasserstoff erfolgt bei einer Temperatur von 0 bis 15°C, vorzugsweise 2 bis 10°C. Das Molverhältnis von Amin zu Fluorwasserstoff beträgt 1:12 bis 1:20, vorzugsweise 1:14 bis 1:18. Besonders gute Ergebnisse werden bei einem Molverhältnis von 1:15 erhalten. Als Reaktionsgefäß dient ein mit einem Rührer versehener Schlaufenreaktor mit innerem

Flüssigkeitsumlauf, dessen Innenzylinder kühlbar ist. Die Reaktanden werden unterhalb des Rührers in den inneren Zylinder des Reaktors eingebracht; der Flüssigkeitsstrom im Innenzylinder ist aufsteigend. Die mittlere Verweilzeit des Reaktionsgemisches im Schlaufenreaktor beträgt 30 bis 90 Minuten.

Die Diazotierung wird bei einer Temperatur von 0 bis 10°C, vorzugsweise 2 bis 8°C durchgeführt. Sie erfolgt mit Hilfe eines Alkalinitrits, insbesondere Natriumnitrit oder Kaliumnitrit. Das Alkalinitrit wird in Pulverform eingesetzt. Das Molverhältnis von Aniliniumfluorid zu Alkalinitrit beträgt 1:1 bis 1:1,15, vorzugsweise 1:1,02 bis 1:1,1; besonders vorteilhaft ist ein Überschuß an Alkalinitrit von 0,03 bis 0,05 mol. Als Reaktionsgefäß dient ein mit einem Rührer versehener Schlaufenreaktor mit innerem Flüssigkeitsumlauf, dessen Innenzylinder und Mantel kühlbar sind. Die Reaktanden werden oberhalb des Rührers in den inneren Zylinder des Reaktors eingebracht; der Flüssigkeitsstrom im Innenzylinder ist absteigend. Die mittlere Verweilzeit des Reaktionsgemisches im Schlaufenreaktor beträgt 30 bis 90 Minuten.

Die Dediazonierung wird bei einer Temperatur von 60 bis 140°C, vorzugsweise 80 bis 110°C durchgeführt. Als Reaktionsgefäß dient ein Rieselfilmreaktor. Dieser besteht im wentlichen aus 1 bis 20, vorzugsweise 3 bis 8, vertikal angeordneten Strömungsrohren, die im oberen Teil von einem Heizmantel und im unterem Teil von einem Kühlmantel umgeben sind. Der Innendurchmesser der Rohre liegt im Bereich von 8 bis 80 mm, vorzugsweise von 20 bis 30 mm; die Länge der Rohre beträgt 100 bis 1000 cm, vorzugsweise 150 bis 400 cm. Die Heizzone beträgt 8 bis 9 Zehntel der jeweiligen Rohrlänge und die Kühlzone 1 bis 2 Zehntel. Das Reaktionsgut läuft über ein Filmerzeugungselement, z.B. einen gezackten Kronenkopf, an der Innenfläche des jewiligen Strömungsrohres herab. Das flüssige Reaktionsprodukt, das Fluorwasserstoff, Alkalifluorid, Wasser und den entstandenen kernfluorierten aromatischen Kohlenwasserstoff enthält, wird in einem Sammelbehälter unterhalb des Reaktors aufgefangen, während der Stickstoff nach oben entweicht. Die organische Phase des Reaktionsproduktes wird in üblicher Weise neutralisiert und dann einer Wasserdampfdestillation unterworfen. Die resultierenden kernfluorierten aromatischen Kohlenwasserstoffe fallen mit einer Ausbeute von mindestens 75 Prozent und mit einer Reinheit von mindestens 98 Prozent (Gaschromatographie) an.

Nach dem erfindungsgemäßen Verfahren sind insbesondere Fluorbenzol und substituierte Fluorbenzole erhältlich. Als Beispiele sind zu nennen Fluorbenzol, 2-Fluortoluol, 2-Fluorethylbenzol, 3-Chlor-fluorbenzol, 3-Brom-fluorbenzol, 3-Methoxy-fluorbenzol, 3-Ethoxy-fluorbenzol, 2,3-Dichlorfluorbenzol, 2,3-Dibrom-fluorbenzol, 3,5-Dichlorfluorbenzol, 3,5-Dibrom-fluorbenzol, 5-Chlor-2-fluortoluol, 2,4-Difluortoluol, 2,3-Difluortoluol, 2-Chlor-6-fluortoluol und 4,4'-Difluordiphenylmethan.

Ein wesentlicher Vorteil des erfindungsgemäßen Verfahrens beruht auf dem Umstand, daß es kontinuierlich durchführbar ist, sicher zu beherrschen ist und die Produktion von Fluorarometen in technischem Maßstab ermöglicht. Hierfür ist die Auswahl der erfindungsgemäß verwendeten Reaktoren von erheblicher Bedeutung.

Die erfindungsgemäß erhaltenen Fluoratomaten dienen als Ausgangsstoffe für die Synthese von phamazeutischen und agrochemischen Wirkstoffen. Insbesondere wird 2-Chlor-6-fluortoluol zur Herstellung eines Antibioticums sowie eines Pesticids zur Bekämpfung des Befalls der Tabakpflanze eingesetzt.

## Beispiel 1

a) In einen Schlaufenreaktor (60 l Inhalt) mit innerem Flüssigkeitsumlauf wurden 45 kg (2,249 kmol) Fluorwasserstoff, der eine Temperatur von 0°C aufwies, durch eine Düse unterhalb des Rührers eingebracht. Durch eine weitere Düse unterhabl des Rührers wurden 21 kg (0,148 kmol) 3-Chlor-2-methylanilin im Laufe von 60 Minuten unter Rühren zugeführt. Die Temperatur des Gemisches wurde dabei auf 5 bis 10°C gehalten; anschließend wurde das Gemisch unter Rühren auf 5°C abgekühlt.

b) Die nach a) erhaltene Salzlösung wurde in einen zweiten Schlaufenreaktor (60 l Inhalt) mit innerem Flüssigkeitsumlauf durch ein Einlaßrohr oberhaf des Rührers überführt. Durch ein weiteres Einlaßrohr oberhalb des Rührers wurden dann 10,6 kg (0,154 kmol) Natriumnitrit-Pulver im Laufe von 1 Stunde unter Rühren zugegeben. Die Temperatur des Gemisches wurde dabei auf 5 bis 10°C grehalten; anschließend wurde das Gemisch unter Rühren auf 5°C abgekühlt.

c) 66 kg der nach b) erhaltenen Lösung von 3-Chlor-2-methyl-benzol-diazoniumfluorid (0,128 kmol) wurdem im Laufe von 100 Minuten kontinuierlich einem Rieselfilmreaktor zugeführt. (Der Rieselfilmreaktor wies 6 Rohre mit einer Länge von jeweils 3,1 m auf; der Innendurchmesser der Rohre betrug 22,3 mm, die Länge der Heizzone 2,5 m und die Länge der Kühlzone 0,6 m. Als Filmerzeugungselement am Säulenkopf diente ein gezackter Kronenkopf. Die Temperatur der Heizzone betrug 100°C, die der Kühlzone 65°C). Das flüssige Reaktionsprodukt wurde in einem Sammelbehälter, der unterhalb des Reaktors angeordnet war und 50 kg wäßrige Flußsäure (45-gewichtsprozentig) enthielt, aufgefangen. Aus dem resultierenden Gemisch wurden nach Abkühlen auf eine Temperatur von 15°C 18 kg organische Phase abgetrennt, und diese wurde mit 5 kg Kalilauge (50-gewichtsprozentig) verrührt. Mit dem so erhaltenen Gemisch wurde eine Wasserdampfdestillation durchgeführt. Die organische Unterphase des Destillats wurde abgetrennt und über Calciumchlorid getrocknet. Es wurden 15,4 kg (0,107 kmol) 2-Chlor-6-fluortoluol (83 Prozent der Theorie, bezogen auf 3-Chlor-2-methylanilin) in einer Reinheit von mehr als 99 Prozent erhalten. Die wäßrige Phase des Destillats enthielt noch 1,3 kg 2-Chlor-6-fluortoluol (7 Prozent der Theorie), so daß die Gesamtausbeute 90 Prozent der Theorie betrug.

Beispiel 2

a) Analog Beispie 1a) wurde diskontinuierlich eine Aniliniumfluorid-Lösung hergestellt; diese Lösung (77 kg) wurde anschließend in den zweiten schlaufenreaktor überführt. — Danach wurde Beispiel 1a) nochmals widerholt, und anschließend wurden Fluorwasserstoff mit einer Menge von 24 kg/h und 3-Chlor-2-methylanilin mit einer Menge von 11,33 kg/h kontinuierlich zugeführt, wobei die Temperatur des Gemisches auf 5°C gehalten wurde.

b) Während der Herstellung der Salzlösung gemäß a) wurde die im zweiten Schalufenreaktor enthaltene Lösung analog Beispie 1b) behandelt; danach wurde Natriumnitrit mit einer Menge von 5,74 kg/h zugeführt und gleichzeitig die gemäß a) synchron erzeugte Aniliniumfluoridlösung kontinuierlich zudosiert. Innerhalb von 33 Stunden wurden 130 kg Diazoniumsalzlösung kontinuierlich hergestellt, wobei die Temperatur auf 5°C gehalten wurde.

c) Analog Beispiel 1 c) wurde die gemäß b) erzeugte Diazoniumsalzlösung kontinuierlich mit einer Menge von 40 kg/h dem Rieselfilmreaktor synchron zugeführt. Die Dediazonierung wurde während einer Zeit von 5 Stunden durchgeführt. Nach dem Aufarbeiten analog Beispiel 1 c) wurden 45,8 kg (0,317 kmol) 2-Chlor-6-fluortoluol in einer Reinheit von mehr als 99 Prozent erhalten (84,5 Prozent der Theorie).

Beispiel 3

Analog Beispiel 1 wurde aus 67 kg (0,184 kmol) Anilin Fluorbenzol hergestellt. Das Molverhältnis Anilin:Fluorwasserstoff:Natriumnitrit betrug 1:10:1,05. — Der hier verwendete Rieselfilmreaktor wies 3 Rohre mit einer Länge von jeweils 2,7 m auf; der Innendurchmesser der Rohre betrug 28,5 mm, die Länge der Heizzone 2,1 m und die Länge der Kühlzone 0,6 m. Die Temperatur der Heizzone betrug 80°C und die der Kühlzone 55°C. — Es wurden nach der Aufarbeitung analog Beispiel 1 c) 13,73 kg (0,143 kmol) Fluorbenzol (77,7 Prozent der Theorie, bezogen auf Anilin) in einer Reinheit von mehr als 99 Prozent erhalten.

**Patentansprüche**

1. Verfahren zur kontinuierlichen Herstellung eines kernfluorierten aromatischen Kohlenwasserstoffs durch Diazotierung eines aromatischen Amins in Gegenwart von Fluorwasserstoff und anschließende Dediazonierung des erhaltenen Aryldiazoniumfluorids in Gengenwart von Fluorwasserstoff, dadurch gekennzeichnet, daß zunächst ein aromatisches Amin in einem Schlaufenreaktor mit wasserfreiem Fluorwasserstoff bei einer Temperatur von 0 bis 15°C zu dem entsprechenden Aniliniumfluorid umgesetzt wird, wobei das Molverhältnis von Amin zu Fluorwasserstoff 1:12 bis 1:20 beträgt, dann das Aniliniumfluorid in einem weiteren Schlaufenreaktor bei einer Temperatur von 0 bis 10°C mit einem Alkalinitrit zu dem entsprechenden Aryldiazoniumfluorid umgesetzt wird, wobei das Molverhältnis von Anilniumfluorid zu Alkalinitrit 1:1 bis 1:1,15 beträgt, und schließlich das Aryldiazoniumfluorid in einem Rieslelfilmreaktor bei einer Temperatur von 60 bis 140°C zu dem entsprechen den kernfluorierten aromatischen Kohlenwasserstoff zersetzt wird.

2. Verfahren nach Anspruch 1, dadurch gekennzeichnet, daß als aromatisches Amin eine Verbindung der Formel

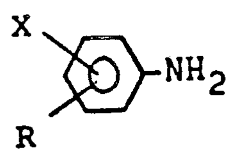

verwendet wird, in der X ein Wasserstoffatom oder ein Halogenatom bedeutet und R einen niederen Alkylrest oder niederen Alkoxyrest oder einen Benzylrest, der gegebenfalls mit einer Aminogruppe substituiert ist, oder X darstellt.

**Revendications**

1. Procédé de fabrication continue d'hydrocarbures aromatiques fluorés sur le cycle par diazotation d'une amine aromatique en présence de fluorure d'hydrogène suivie d'une dédiazonation (décomposition) du fluorure d'aryldiazonium formé en présence de fluorure d'hydrogène, procédé caractérisé en ce que l'on commence par fair régir une amine aromatique, dans un réacteur à colonne d'écoulement en boucles, avec du fluorure d'hydrogène anhydre à une température de 0 à 15°C pour former le fluorure d'anilinium correspondant, le rapport molaire de l'amine au fluorure d'hydrogène étant compris entre 1:12 et 1:20, puis on fait réagir ce fluorure d'anilinium dans un autre réacteur à colonne d'écoulement en boucles, à une température de 0 à 10°C, avec un nitrite de métal alcalin pour obtenir le fluorure d'aryldiazonium correspondant, le rapport molaire de fluorure d'anilinium au nitrite de métal alcalin étant compris entre 1:1 et 1:1,15, et enfin, on décompose le fluorure d'aryldiazonium dans un réacteur à pellicule ruisselante à une température de 60 à 140°'C, ce qui donne l'hydrocarbure aromatique fluoré sur le noyau.

2. Procédé selon la revendication 1, caractérisé en ce que l'amine aromatique est un composé de formule:

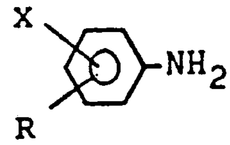

dans laquelle X représente un atome d'hydrogène 'ou d'halogène et R un alkyle inférieur ou alcoxy inférieur ou un radical benzyle éventuellement porteur d'un groupe amino, ou bien R a les significations indiquées pour X.

**Claims**

1. A process for the continuous preparation of a nuclear-fluorinated aromatic hydrocarbon by diazotization of an aromatic amine in the presence of hydrogen fluoride and subsequent dediazoniation of the resulting aryldiazonium fluoride in the presence of hydrogen fluoride, characterized in that first an aromatic amine is reacted in a loop reactor with anhydrous hydrogen fluoride at a temperature of 0 to 15°C to give the corresponding anilinium fluoride, the molar ratio of amine to hydrogen fluoride being 1:12 to 1:20, then the anilinium fluoride is reacted in a second loop reactor, at a temperature of 0 to 10°C, with an alkali metal nitrite to give a corresponding aryldiazonium fluoride, the molar ratio of anilinium fluoride to alkali metal nitrite being 1:1 to 1:1.15 and finally the aryldiazonium fluoride is decomposed in a falling film reactor at a temperature of 60 to 140°C to give the corresponding nuclear-fluorinated aromatic hydrocarbon.

2. The process as claimed in claim 1, characterized in that the aromatic amine used is a compound of the formula

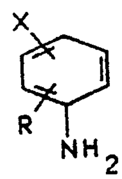

in which X denotes a hydrogen atom or a halogen atom, and R represents a lower alkyl radical or lower alkoxy radical, or a benzyl radical which is optionally substituted by an amino group, or represents X.